# EUROPEAN PATENT SPECIFICATION

(11) **EP 3 560 524 B1**
(45) Date of publication and mention of the grant of the patent: **12.01.2022**
(21) Application number: 17885345.3
(22) Date of filing: 18.04.2017
(51) Int. Cl.: A61L 24/08, A61L 24/04, A61L 24/06, C08J 9/26, C08F 291/08, C08F 220/06, C08F 220/56

(54) **DRY GEL SPONGE OF SUPER WATER-ABSORBENT POLYMER HYDROGEL, PREPARATION METHOD THEREFOR AND USE THEREOF**
TROCKENGELSCHWAMM EINES SUPERWASSERABSORBIERENDEN POLYMERHYDROGELS, HERSTELLUNGSVERFAHREN DAFÜR UND VERWENDUNG DAVON
ÉPONGE DE GEL SEC D'UN HYDROGEL POLYMÈRE SUPER-HYDROABSORBANT, PROCÉDÉ DE PRÉPARATION S'Y RAPPORTANT ET UTILISATION ASSOCIÉE

(30) Priority: 20.12.2016 CN 201611188263
(43) Date of publication of application: 30.10.2019
(73) Proprietor: Solaplus Biotechnology Co., Ltd, WenZhou, Zhejiang 325000 (CN)
(72) Inventor: GUO, Ximin, WenZhou Zhejiang 325000 (CN); QIAN, Zhiyong, WenZhou Zhejiang 325000 (CN); TUO, Xiaoye, WenZhou Zhejiang 325000 (CN)
(74) Representative: Cabinet Beau de Loménie
(86) International application number: PCT/CN2017/080848
(87) International publication number: WO 2018/113147

(56) References cited:
- CN-A- 101 564 557
- CN-A- 101 564 557
- CN-A- 101 613 512
- CN-A- 101 654 537
- CN-A- 101 654 537
- CN-A- 104 558 988
- CN-A- 104 558 988
- CN-B- 101 613 512
- US-A1- 2005 084 512

## Description

### FIELD OF THE INVENTION

The invention relates to a biological material for emergency hemostasis, and in particular to a superabsorbent polymer xerogel sponge for emergency hemostasis.

### BACKGROUND ART OF THE INVENTION

Hemostasis is an important part of emergency medical rescue, especially in the war and wound rescue caused by harsh war environment and complex emergencies, it is particularly important that hemostasis is made rapidly and effectively. Different from general hemostatic agents used in clinical practice at ordinary times, emergency hemostatic agents are applied in a more urgent timing, with operators not professional rescue personnel in most cases, thereby being more demanding on their portability, usability, and even preservation. In such case, an ideal emergency hemostatic agent meets the following conditions simultaneously: 1. rapid hemostatic effect on aorta and vein may be achieved within 2 min. 2. easy to use whenever and wherever, without need to mix or other special preparation before using. 3. simple to use, without requiring special training, convenient for the war wound, trauma personnel and first aid team for self-rescue and mutual rescue in complex emergencies. 4. portable and durable with long preservation time. 5. preservation for more than 2 years at room temperature, and a few weeks or longer preserved in some special conditions (-10°C-55°C). 6. safe to use, without causing toxicity to tissue or cells, secondary damage to local tissue, distal body vessel embolism, or bacterial or viral infection. 7. convenient for mass production with low production cost. [E.M. Acheson, B.S. Kheirabadi, R. Deguzman, E.J. Jr Dick, J.B. Holcomb. J. Trauma. 2005,59(4),865-74.]

Currently the hemostatic materials on the market mainly comprise fibrin glue, collagen (e.g. gelatin sponge), multi-micro porous inorganic materials (e.g. zeolite), starch (e.g. Trauma DEX), carboxymethyl cellulose (e.g. soluble stanching gauze), α-cyanoacrylate tissue glue and the like, which have satisfactory hemostatic effect in hospital yet obvious shortcomings in self-rescue and mutual rescue. For example, fibrin glue is obtained from blood, which implies a high cost and easily spreads disease; collagen relies solely on activating blood platelets to stop bleeding which has limited hemostatic effect and has poor mechanical strength and tissue adhesion; porous zeolite and Trauma DEX releasing a lot of heat energy when absorbing water from blood, resulting in tissue burns; soluble gauze of carboxymethyl cellulose is incapable of degrading in wound surface, and has limited hemostatic ability.

Chitosan is a natural polycationic polysaccharide made from chitin by deacetylation. It has many functions such as coagulation, bacteriostasis, promoting wound healing and inhibiting scar formation. As a multi-functional material with good biocompatibility, no immunogenicity, and no irritation, it is recognized as a GRAS substance by the FDA of United States in 2001 (general recognized as safe). The chitosan-based rapid hemostatic agent refers to a series of new multi-effect hemostatic materials prepared by physically and chemically modifying chitosan and its derivatives and compounding with other functional materials to improve its hemostatic effect. In recent years, the rapid hemostatic agent with chitosan and its derivatives as the main hemostatic materials has been increasingly extensively used in the field of hemostasis, and yet still has some shortcomings. For example, the HemCon chitosan hemostatic sponge (HC) produced by HemCon Corporation in the United States can significantly increase the survival rate of animals with v-grade liver injury and reduce blood loss, yet in the femoral artery injury model, HC fails to make continuous and complete hemostasis as expected; Celox hemostatic powder mainly made from Celox, a granular mixture extracted from shrimp shells, containing chitosan, has remarkable hemostatic effect, yet the granular substance will stick to the wound surface, which may increase the difficulty of the later treatment of the wound.

Studies in recent years have found that water absorption and concentration can rapidly initiate blood coagulation and significantly promote the process of blood coagulation. Once the water in blood is absorbed by materials, the concentration of red blood cells, platelets, clotting factors and other factors will be increased, which promotes the blood coagulation. For example, zeolite hemostatic granules absorb water in blood into the pores of the materials through the action of molecular sieve, so as to achieve blood coagulation. Further, the combination of charge adsorption of chitosan with molecular sieves of carboxyl-rich macromolecules such as polyacrylic acid is also a new method to develop new hemostatic materials. Sodium polyacrylate hemostatic powder (ZL 03153260.8) and carboxymethyl cellulose grafted polyacrylic acid (ZL 200410096825.4) prepared by Dou Guifang in China can achieve blood coagulation by absorbing water in blood and concentrating blood with superabsorbent polymer material. The hemostatic sponge prepared by Guo Ximin also showed a good hemostatic effect by compounding sodium polyacrylate grafted chitosan, gelatin and chitosan (ZL 201110044474.2). As limited by factors such as process and dosage form, the inventions fail to perform an ideal hemostatic effect, though the significant coagulation effect made by simple water absorption and concentration is shown in the inventions. As to materials provided by patent ZL 03153260.8 and patent ZL 200410096825.4, since sodium polyacrylate and polyacrylamide superabsorbent molecules are not biodegradable, the powder prepared is easily flushed away by blood flow and quickly saturated when subject to arterial or venous injury with rapid bleeding, and has limited hemostatic effect and is difficult to clean up after using; the superabsorbent sodium polyacrylate grafted chitosan in the material provided in patent ZL 201110044474.2 only serves as one component of the material, the overall water absorption effect of the material is limited. The superabsorbent polymer modified macromolecules provided in the patents cited above fail to solve the problem of dosage form, the typical forms thereof are powder or powder doping, which not only limit the function of the material as a whole, but also cause problems of cleaning and in-vivo residues.

CN 104 558 988 A discloses a chitosan sponge prepared by blending an acid solution of chitosan with an aqueous solution of PVA , adding sulfuric acid, PEG, 2-acrylamide-2-methyl propanesulfonic acid, glycerin, glutaraldehyde and a pore forming agent (sodium hydrogencarbonate) at 50 to 70°C, uniformly stirring and then pouring into a mold and drying at room temperature to obtain the chitosan sponge.

### SUMMARY OF THE INVENTION

In view of the shortcomings of the emergency hemostatic products on the market, the invention provides a chitosan hemostatic sponge with flexible molecular cross-linking and molecular modification of superabsorbent polymer - a superabsorbent polymer xerogel sponge. The sponge can be prepared by a one-pot method, which is convenient for large-scale production, and the material has good integrity and flexibility, which can lock water through chemical bonds after absorbing water and still maintain integrity under pressure. According to the material, through rapid and strong water absorption capacity, stable blood clots can be rapidly formed on the surface of the material and the damaged part of blood vessels, so as to achieve rapid hemostasis.

To achieve the said purpose, the invention provides a A-superabsorbent polymer xerogel sponge is a three-dimensional network porous sponge with chitosan as its skeleton, superabsorbent polymer as its branched chain, and macromolecule or polymer with flexible structure as its cross-linking agent; wherein the superabsorbent polymer is sodium polyacrylate, polyacrylamide, methyl methacrylate and/or polyvinyl alcohol, and the cross-linking agent is one or more of methacrylic anhydride-terminated polyethylene glycol, acrylic anhydride-terminated polyethylene glycol, maleic anhydride-terminated polyethylene glycol, itaconic anhydride-terminated polyethylene glycol, according to claim 1.

In the superabsorbent polymer xerogel sponge described above, preferably, the chitosan accounts for 1~10% of the total mass of the sponge; the superabsorbent polymer accounts for 85-98% of the total mass of the sponge; and the cross-linking agent accounts for 0.5∼5% of the total mass of the sponge.

The superabsorbent polymer xerogel sponge described above, preferably further includes plasticizer, emulsifier and/or antioxidant.

In the superabsorbent polymer xerogel sponge described above, preferably, the plasticizer is selected from propylene glycol, glycerin, sorbitol, span, tween, polyethylene glycol and/or sodium fatty acid.

The invention provides also a preparation method of a superabsorbent polymer xerogel sponge according to claim 7.

The preparation method described above preferably comprises the following steps: adding the cross-linking agent and the chitosan solution with a concentration of 1%-30% (g/ml) into the superabsorbent polymer monomer aqueous solution with a concentration of (70-99) v% at room temperature, stirring, then adding the ammonium persulfate, after stirring, adding the pore-forming agent with a concentration of 1%-20% (g/ml), stirring and reacting at 50-100°C for 30-60 minutes to obtain porous sponge.

In the preparation method described above, preferably, a plasticizer is added into the porous sponge, the plasticizer is glycerin with a concentration of 1wt%-10wt%, propylene glycol with a concentration of 1wt%-5wt% or sorbitol with a concentration of 1wt%-5wt%.

In the preparation method described above, preferably, a emulsifier is added into the foaming reaction system, the emulsifier is span with a concentration of 1wt%-10wt%, tween with a concentration of 1wt%-10wt% or SDS solution with a concentration of 1wt%-10wt%.

In a further aspect, the invention provides a superabsorbent polymer xerogel sponge prepared by the preparation method described above.

In a yet further aspect, the invention provides a superabsorbent polymer xerogel sponge for use as a hemostatic sponge, preferably in emergency hemostasis, packing hemostasis, and wound plugging.

In the superabsorbent polymer xerogel sponge and the preparation method thereof, preferably, the polyethylene glycol is selected from PEG-400, PEG-600, PEG-1500, PEG-4000, PEG-6000 and PEG-20000.

The hemostatic sponge provided by the invention is a three-dimensional network porous sponge formed by chitosan as skeleton, superabsorbent polymer as branched chain, macromolecule or polymer with flexible structure as cross-linking agent, Na₂CO₃ or NaHCO₃ as pore-forming agent, with the pore size of 500nm-20µm. The invention has the beneficial effects as follows:
(1) The xerogel sponge prepared by the invention is instantaneously superabsorbent. It may instantly absorb water after touching it, with the water absorption rate of 60-600 times of the dead weight. Through rich hydroxyl groups, the sponge is combined with water molecules by hydrogen bonds, so as to have intramolecular water locking property. The absorbed water cannot be squeezed out by external forces.
(2) The sodium polyacrylate grafted with the chitosan in the sponge has a super-hydrophilic effect, which causes swelling the material to narrow down the pore passage and reduce blood flow velocity, and highly concentrating the hemostatic components such as platelets, thrombin, fibrin and the like in blood, when contacted with blood, thereby accelerating and strengthening the formation of blood clots.
(3) The chitosan has an aggregation (agglutination) effect on red blood cells having negative charges through positive charges carried thereby in the blood coagulation process. The chitosan can mediate adhesion of platelets, with the formed chitosan/platelet composite accelerating polymerization of fibrin monomers and jointly forming clots. Moreover, the chitosan can also stimulate platelet adsorption, making platelets converted from a static phase to a functional phase, then the platelets aggregated on the chitosan quickly forming thrombi to close wounds and achieve hemostasis. Further, a large amount of serum proteins can be absorbed by the chitosan once it is contacted with blood. The denaturation and activation of the proteins adsorbed on the surface of the chitosan can start an exogenous coagulation pathway. Also, the chitosan has the effects of inhibiting the fibrinolytic activity in vivo and reducing the capability of macrophages to secrete plasminogen activator, so as to reduce the dissolution of fibrin and improve hemostatic effect.
(4) Using polyethylene glycol as the cross-linking agent may significantly improve the mechanical property of the material, make the hemostatic sponge soft and elastic and turn into a gelatin after water absorption, so as to maintain integrity and mechanical strength, and is convenient for external pressure.
(5) The hemostatic sponge can be tightly attached to the wound surface to close the wound surface, and prevent bacteria and harmful particles in the environment from contacting the wound surface. Also, the hemostatic sponge is yet breathable and permeable, and will not adhere to the tissues of the wound surface.
(6) The xerogel sponge prepared by the invention can be used for emergency hemostasis, especially for bleeding caused by large arterial and venous injuries. The xerogel sponge provided by the invention has a significant effect of water absorption and expansion, thereby being available for packing hemostasis to stop confined space bleeding, such as nasal bleeding and ballistic injury. Also the hemostatic sponge has the characteristics of tissue adhesion, so as to be used for wound plugging, such as rupture of stomach, pancreas, bladder, uterus, liver, kidney and the like.
(7) The hemostatic sponge of the invention can be prepared by a one-pot method without separation and purification process in the course, which saves cost, facilitates quality control and is beneficial to mass production.

### BRIEF DESCRIPTION OF THE DRAWINGS

Figure 1 illustrates the structure of a superabsorbent polymer xerogel sponge.
Figure 2 illustrates the water absorbency of the superabsorbent polymer xerogel sponge.
Figure 3 illustrates the hemostatic effect of femoral artery in rabbits.

### DETAILED DESCRIPTION OF THE INVENTION

The invention is further illustrated with reference to specific embodiments as follows. It should be noted that the embodiments herein are used only to illustrate the invention but not to limit the scope of the invention.

### Embodiment 1 Preparation of Sample 1 of the superabsorbent polymer xerogel sponge

A polyethylene glycol (PEG) modified by methacrylic anhydride (MAA) as cross-linking agent and a sodium polyacrylate as water-absorbing polymer are reacted with MAA-terminated chitosan (MAACTS) to prepare sodium polyacrylate modified chitosan. The flow chart of the synthesis process is as follows:

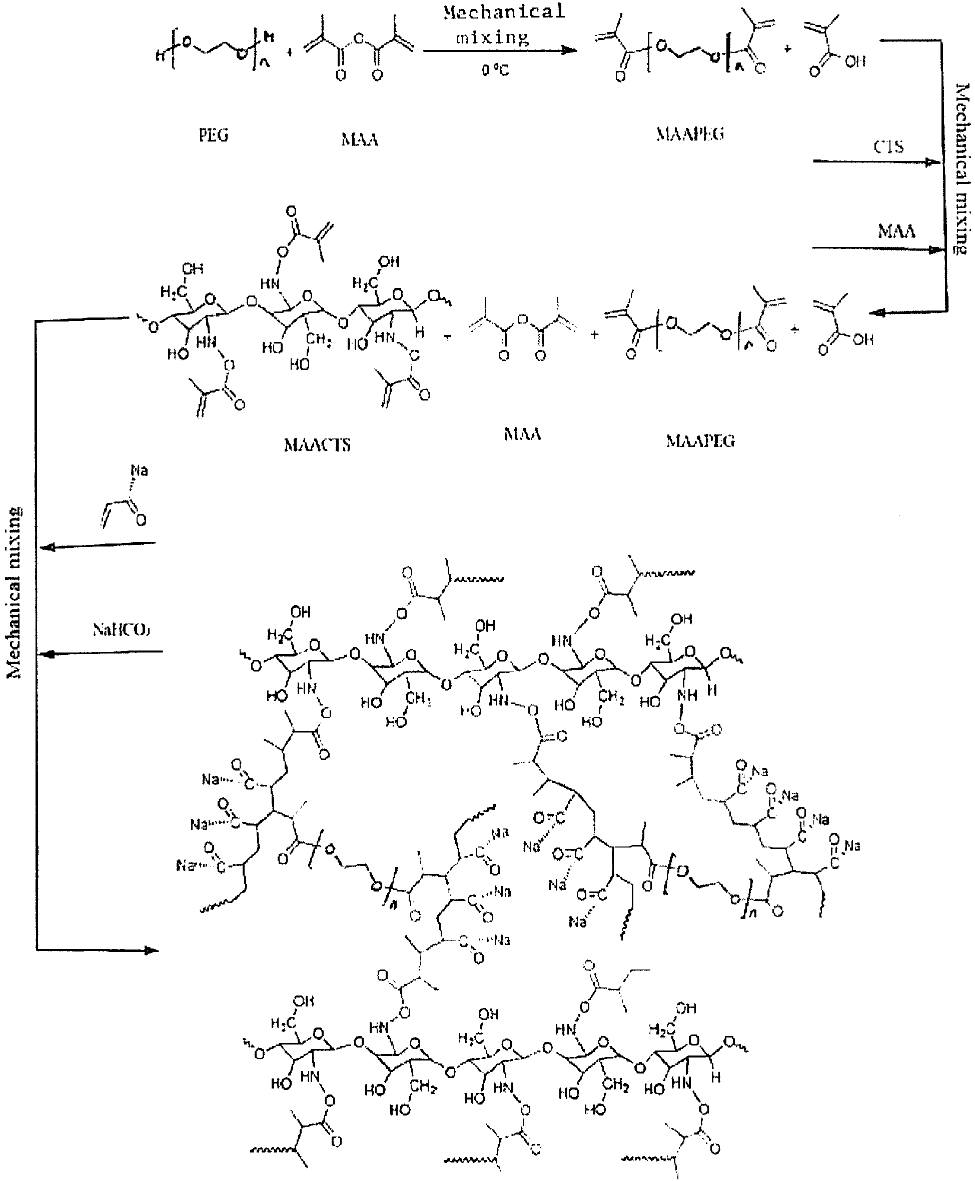

Step 1: To 100 ml of chitosan (CTS) solution with a mass/volume fraction (g/ml) of 3%, 3 ml of methacrylic anhydride (MAA) was added. The mixture was mechanically stirred for 60 min at a speed of 1200 r/min to give methacrylic anhydride-terminated chitosan (MAACTS).

Step 2: To 50 ml of chloroform, 1 g of PEG-6000 was added. The mixture was magnetically stirred for 3 h, then 1 ml of methacrylic anhydride was added and magnetically stirred for 6 h, and then volatilized to remove chloroform to give PEG-6000 terminated with methacrylic acid white powder.

Step 3: To 300 ml of deionized water in a salt ice bath, 100 g of NaOH was added, the solution was mechanically stirred.When the temperature of the NaOH solution was droped to 0°C, 200 ml of acrylic acid (AA) solution was added while the solution temperature was controlled within 80°C. Then the solution temperature drops to room temperature, the solution obtained in Step 1 and the white powder obtained in Step 2 were added, and the mixture was mechanically stirred for 4 h.

Step 4: To the mixture obtained in Step 3, 6 g of ammonium persulfate was added, the mixture was mechanically stirred for 30 min, then 100 ml of 10% NaHCO₃ was added with mechanically stirring to give white emulsion. The emulsion was added into a teflon grinding tool for reacting for 120 min at 80°C to give white porous sponge.

Step 5: The sponge obtained in Step 4 was sequentially washed in 60%, 70% and 80% ethanol solutions for 4 times, and then volatilized to remove the ethanol to give white elastic sponge. The sponge was packaged, cut, and irradiated by cobalt 60 for sterilization.

### Embodiment 2 Preparation of Sample 2 of the superabsorbent polymer xerogel sponge

Step 1: To 100 ml of chitosan solution with a mass/volume fraction (g/ml) of 3%, 3ml of methacrylic anhydride was added. The mixture was mechanically stirred for 60 min at a speed of 1200 r/min to give methacrylic anhydride-terminated chitosan.

Step 2: To 50 ml of chloroform, 1 g of PEG-20000 was added. The mixture was magnetically stirred for 3 h, then 1 g of maleic anhydride was added and magnetically stirred for 6 h. Then the solution was added dropwise into excess ether to obtain white powder. The powder was dried under vacuum for 36 h. The dried white powder was purified in a co-precipitation system of chloroform/ether for 4 times, and then dried under vacuum for 36 h to give PEG-20000 terminated with maleic anhydride white powder.

Step 3: To 150ml of deionized water in a salt ice bath, 50 g of NaOH was added, the solution was mechanically stirred. When the temperature of the NaOH solution drops to 0°C, 100 ml of acrylic acid solution was added while the solution temperature was controlled within 80°C. Then the solution temperature drops to room temperature, 50 g of acrylamide, the solution obtained in Step 1 and the white powder obtained in Step 2 was added, and the miature was mechanically stirred for 4 h.

Step 4: To the mixture obtained in Step 3, 6 g of ammonium persulfate was added, the mixture was mechanically stirred for 30 min, then 100 ml of 10% NaHCO₃ was added with mechanically stirring to give white emulsion. The emulsion was added into a teflon grinding tool for reacting for 120 min at 80°C.The product was sequentially washed in 60%, 70% and 80% ethanol solutions for four times, then volatilized to remove the ethanol to give white porous sponge. The sponge was packaged, cut, and irradiated by cobalt 60 for sterilization.

### Embodiment 3 Preparation of Sample 3 of the superabsorbent polymer xerogel sponge

Step 1: To 100 ml of chitosan solution with a mass/volume fraction (g/ml) of 3%, 3 ml of methacrylic anhydride was added. The mixture was mechanically stirred for 60 min at a speed of 1200 r/min to give methacrylic anhydride-terminated chitosan.

Step 2: To 50 ml of chloroform, 1g of PEG-20000 was added. The mixture was magnetically stirred for 3 h, then 1 g of itaconic anhydride was added and magnetically stirred for 6 h. The solution was added dropwise into excess ether to obtain white powder. The powder was dried under vacuum for 36 h. The dried white powder was purified in a co-precipitation system of chloroform/ ether for 4 times, and then dried under vacuum for 36 h to give PEG-20000 terminated with itaconic anhydride white powder.

Step 3: To 150ml of deionized water in a salt ice bath, 50 g of NaOH was added, the mixture was mechanically stirred. When the temperature of the NaOH solution was droped to 0°C, 100 ml of acrylic acid solution was added while the solution temperature was controlled within 80°C. Then the solution temperature drops to room temperature, 50 g of acrylamide, 50 ml of methyl methacrylate solution, the solution obtained in Step 1 and the white powder obtained in Step 2 was added, and the mixture was mechanically stirred for 4 h.

Step 4: To the mixture of Step 3, 6 g of ammonium persulfate, the mixture was mechanically stirred for 30 min, then 100 ml of 10% NaHCO₃ was added with mechanically stirring to give white emulsion. The emulsion was added into a teflon grinding tool for reacting for 120 min at 80°C to give white porous sponge.

Step 5: The sponge obtained in Step 4 was sequentially washed in 60%, 70% and 80% ethanol solutions for four times, and then volatilized to remove ethanol to give white porous sponge. The sponge was packaged, cut, and irradiated by cobalt 60 for sterilization.

### Embodiment 4 Preparation of Sample 4 of the superabsorbent polymer xerogel sponge

Step 1: To 100 ml of chitosan solution with a mass/volume fraction (g/ml) of 3%, 3ml of methacrylic anhydride was added. The mixture is mechanically stirred for 60 min at a speed of 1200 r/min to give methacrylic anhydride- terminated chitosan.

Step 2: To 50 ml of chloroform, 1 g of PEG-6000 was added. The mixture was magnetically stirred for 3 h, then 1 ml of methacrylic anhydride was added and magnetically stirred for 6 h, and then volatilized to remove chloroform to give PEG-6000 terminated with methacrylic acid white powder.

Step 3: To 150 ml of deionized water in a salt ice bath, 50 g of NaOH was added, the solution was mechanically stirred. When the temperature of the NaOH solution was dropped to 0°C, 100 ml of acrylic acid solution was added while the solution temperature was controlled within 80°C. Then the solution temperature drops to room temperature, 50 g of acrylamide, 50 ml of polyvinyl alcohol solution, 50 ml of methyl methacrylate solution, the solution obtained in Step 1 and the white powder obtained in Step 2 was added, and the mixture was mechanically stirred for 4 h.

Step 4: To the mixture obtained in Step 3, 6 g of ammonium persulfate was added, the mixture was mechanically stirred for 30 min, then 100 ml of 10% NaHCO₃ was added with mechanically stirring to give white emulsion. The emulsion was added into a teflon grinding tool for reacting for 120 min at 80°C to give white porous sponge.

Step 5: The sponge obtained in Step 4 was sequentially washed in 60%, 70% and 80% ethanol solutions for four times, and then volatilized to remove ethanol to give white porous sponge. The sponge was packaged, cut, and irradiated by cobalt 60 for sterilization.

### Embodiment 5 Physical and Structural Characteristics

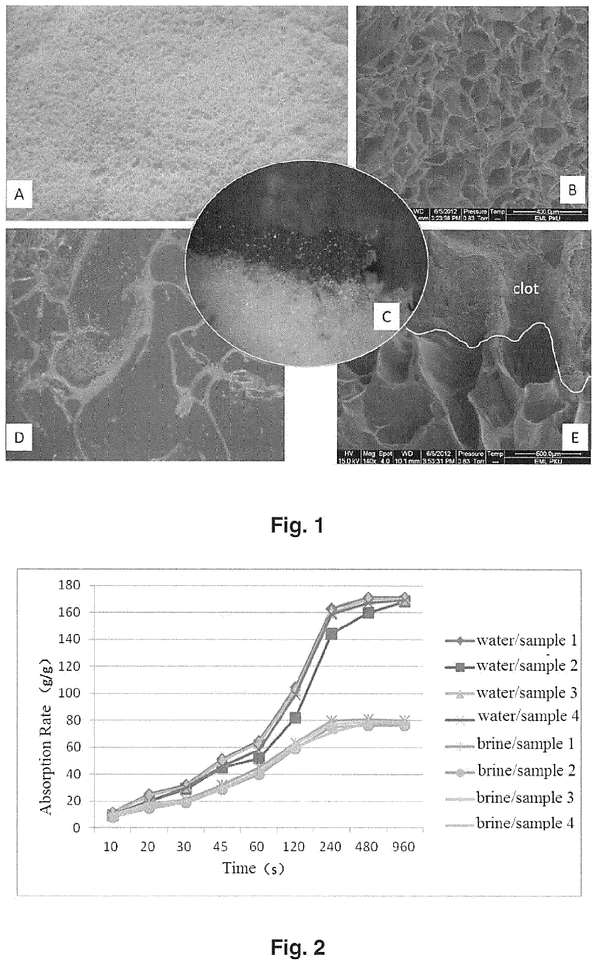

The emergency hemostatic sponge material obtained in the embodiments 1-4 has a porous sponge-like structure (FIG. 2A), with a porosity of 40%-80%, a pore size of 100 um - 2 mm, and extensive communication between the pores. Blood was rapidly drawn into the porous structure and concentrated once the sponge contacting with blood. After water absorption, the pore wall was thicken and gelled, the lumen was narrow down, and the viscoelasticity of the wall was increased, thereby buffering the blood flow. Moreover, the blood was high concentrated, rapidly forming and starting clotting, the formed blood clots would become more firmer through continuous water absorption, thereby a quick and effective hemostasis effect was achieved.

The samples of the superabsorbent polymer xerogel sponge prepared by the embodiments 1-4 were observed for their general structures before and after contacting with blood, and the microstructures were examined with a scanning electron microscope. Represented by the Sample 1 as shown in Figure 1, the samples are soft porous sponges with elastic, flexible and odorless features (FIG. 1 A). An interconnected porous structure is displayed under an electron microscope (FIG. 1B). Upon contacting with blood (FIG.1C), the pore wall rapidly absorbs water and expands, making the channels narrow down until closed (FIG.1D), and blood coagulate for rapid concentration (FIG.1E).

### Embodiment 6 Water-absorbing Characteristic

Water-absorbing is an important characteristic in the invention. 0.100 g of a sample was accurately weighed and placed in a nylon fabric bag, which was then immersed in distilled water (pH = 7.0, and the temperature of 25°C) for naturally absorbing water and swelling at room temperature. The swelling sample was weighed after absorbing water on the surface of the fabric bag at 10 seconds, 20 seconds, 30 seconds, 45 seconds, 1 minute, 2 minutes, 4 minutes and 8 minutes, 16 minutes and 32 minutes respectively. The water absorption rate at each time point was calculated, so as to draw the relation curve between water absorption rate and time.

The formula for calculating the water absorption rate Q is that: Q= (m2-m1)/m1, wherein, Q is the water (brine) absorption rate, g/g; m1 is the mass of the sample before imbibing, g; m2 is the mass of the sample after imbibing, g.

Results as shown in Figure 2 shows that the samples 1-4 exhibit similar water absorption, reach maximum water absorption rate of about 165 times and maximum brine absorption rate of about 72 times within 3 minutes.

### Embodiment 7 In-vitro Clotting Ability

The emergency hemostatic sponge prepared in Embodiment 1 was used for in vitro coagulation test. Collagen hemostatic fiber (Avitene) was used as a control group to be compared with the material provided by the invention. Blood samples were collected from healthy donors (n=3). The experimental process and results as follows:
Effect analysis of in-vitro coagulation test is that:
The in-vitro clotting ability of the emergency hemostatic sponge was detected by a hemate elastometer (TEG) (as shown in Table 1). The experimental results show that compared with the negative control group, R-time and K-time of the samples in each group are significantly decreased (p < 0.05), while Angle deg and MA values are significantly increased; compared with the positive control group, no significant difference is provided in R-time, K-time, Angle deg and MA (p > 0.05). These indicate that the experimental materials in each group have the same strength as the collagen fiber (Avitene) as the control group to initiate the endogenous coagulation mechanism of blood.

**Table 1 TEG Analysis**

| *Materials* | *R^{c}(min)* | *K^{d} (min)* | *Angle deg^{e}* | *MA (mm)^{f}* |
|---|---|---|---|---|
| Negative control group^{a} | 7.63 ± .45 | 3.80 ± 0.22 | 51.47 ± 1.52 | 52.20 ± 2.41 |
| Positive control group^{b} | 3.41 ± 0.24 | 2.63 ± 0.33 | 57.33 ± 0.71 | 54.51 ± 2.41 |
| Sample 1 | 3.56 ± 0.25 | 2.23 ± 0.28 | 60.50 ± 0.65 | 59.70 ± 1.45 |
| Sample 2 | 3.35 ± 0.32 | 2.22 ± 0.19 | 59.46 ± 0.48 | 58.37 ± 2.26 |
| Sample 3 | 3.64 ± 0.54 | 2.43 ± 0.22 | 58.47 ± 0.57 | 57.64 ± 2.19 |
| Sample 4 | 3.55 ± 0.19 | 2.59 ± 0.31 | 56.78 ± 0.39 | 59.34 ± 1.98 |

| | | | | |
|---|---|---|---|---|
| a No hemostatic material is added to the negative control group, and blood is naturally coagulated. b The positive control group is collagen sponge (Avitene), a strong trigger for endogenous coagulation. c *R*: the time that activating thrombin should be used, namely, the time that blood clots just occurred in blood. d *K*: the time required to form a stable clot. e *Angle deg:* measuring the rate of fibrin netting, thereby the rate of clot formation is evaluated. f *MA:* the strength of the fibrin blood clot. | | | | |

### Embodiment 8 Hemostasis test of femoral artery of rabbits

Healthy male rabbits, weighing 2.5 - 3.1 kg, are anesthetized with 1% of pentobarbital sodium in the marginal ear vein. The groin skin of right hind leg was shaved, the skin and subcutaneous tissue were cut, the femoral artery 2cm from the groin was separated, and then punctured through the wall of the contralateral blood vessel with a 16G needle. Then the arterial clamp was released for naturally bleeding for 5 seconds. Using gauze to wipe off the blood, and then immediately stop bleeding. Common gauze of 32 layers was used as a negative control, and QuikClot Combat Gauze (Z-Medica, U.S., Army Equipment Force) was used as a positive control. Each group comprises 10 animals. Local pressure was applied for 1 minute to observe whether the bleeding was stopped. The material was carefully removed after five minutes if no bleeding, and then observed whether bleeding. The results are shown in Table 2.

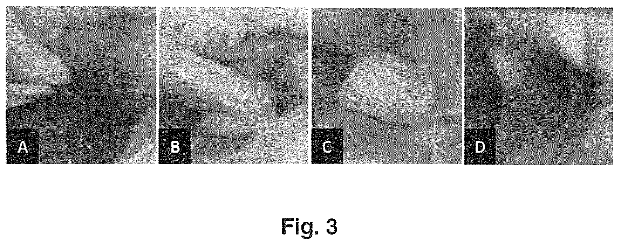

In the course of hemostasis, no effective hemostasis was shown in the negative control group, and all animals died. In QuikClot Combat Gauze group, 7 animals still had different degrees of bleeding after removing pressure, only 3 animals showed no bleeding yet all bled again after 5 minutes of material removal. The water-absorbent polymer xerogel sponge of the four formulas provided by the invention achieved hemostatic effect in the hemostasis of rabbit femoral artery in all cases (Figure 3). All animals in the experimental groups had no bleeding within 5 minutes after removing pressure. The hemostatic sponge closely adhered to the wound tissue once removed from the wound surface, showing excellent wound closure. After the removal, the wound surface was clean, no secondary bleeding, and no blood clot residue. The bleeding loss of animals was far less than that of the control group (p<0.01).

**Table 2 Hemostasis of Femoral Artery in Rabbits**

| Performance | | | | | |
|---|---|---|---|---|---|
| Materials | Number of animals | Press-time | Successful hemostasis ^{d} | Secondary bleeding^{e} | Blood loss (g) |
| Control 1 ^{a} | 10 | 1 | 0/10 | NA | NA |
| Control 2 ^{b} | 10 | 1 | 5/10 | 3/10 | 4.67±0.31 |
| Control 2 ^{c} | 10 | 1 | 7/10 | 2/10 | 0.39±0.57 |
| Sample 1 | 10 | 1 | 10/10 | 0/10 | 2.35±0.21 |
| Sample 2 | 10 | 1 | 10/10 | 0/10 | 3.24±0.35 |
| Sample 3 | 10 | 1 | 10/10 | 0/10 | 2.69±0.19 |
| Sample 4 | 10 | 1 | 10/10 | 0/10 | 2.89±0.36 |

| | | | | | |
|---|---|---|---|---|---|
| a Control 1 is 32 layers of plain gauze b Control 2 is QuikClot Combat Gauze C Control 3 is Celox d No bleeding after removal of pressure is considered as successful hemostasis e No bleeding after the removal of pressure yet bleeding within the next 5 minutes is considered secondary bleeding. | | | | | |

## Claims

1. A superabsorbent polymer xerogel sponge, having a three-dimensional network porous sponge with chitosan as its skeleton, superabsorbent polymer as its branched chain, and macromolecule or polymer with flexible structure as its cross-linking agent, wherein:
the superabsorbent polymer is sodium polyacrylate, polyacrylamide, polymethyl methacrylate and/or polyvinyl alcohol;
the cross-linking agent is one or more of: methacrylic anhydride-terminated polyethylene glycol, acrylic anhydride-terminated polyethylene glycol, maleic anhydride-terminated polyethylene glycol, and itaconic anhydride-terminated polyethylene glycol; and
the chitosan is one or more of a methacrylic anhydride-terminated chitosan which is modified with methacrylic anhydride, an acrylic anhydride-terminated chitosan which is modified with acrylic anhydride, a maleic anhydride-terminated chitosan which is modified with maleic anhydride and an itaconic anhydride-terminated chitosan which is modified with itaconic anhydride.

2. The superabsorbent polymer xerogel sponge according to claim 1, wherein the polyethylene glycol is selected from PEG-400, PEG-600, PEG-1500, PEG-4000, PEG-6000 and PEG-20000.

3. The superabsorbent polymer xerogel sponge according to claim 1 or 2, wherein the chitosan accounts for 1~10% of the total mass of the sponge; the superabsorbent polymer accounts for 85~98% of the total mass of the sponge; the cross-linking agent accounts for 0.5~5% of the total mass of the sponge.

4. The superabsorbent polymer xerogel sponge according to claim 1 or 2, wherein the sponge further includes plasticizer, emulsifier and/or antioxidant.

5. The superabsorbent polymer xerogel sponge according to claim 4, wherein the plasticizer is selected from propylene glycol, glycerin, sorbitol, span, tween, polyethylene glycol and/or sodium fatty acid.

6. The superabsorbent polymer xerogel sponge according to claim 4, wherein the emulsifier is selected from span, tween or SDS solution with a concentration of 1wt%-10wt%.

7. A preparation method of a superabsorbent polymer xerogel sponge, wherein the method comprises: mixing chitosan, superabsorbent polymer monomer, cross-linking agent, ammonium persulfate and pore-forming agent, and then obtaining porous sponge through free radical polymerization reaction; wherein,
the superabsorbent polymer monomer is sodium acrylate, acrylamide, methyl methacrylate and/or vinyl alcohol;
the cross-linking agent is one or more of: methacrylic anhydride-terminated polyethylene glycol, acrylic anhydride-terminated polyethylene glycol, maleic anhydride-terminated polyethylene glycol, itaconic anhydride-terminated polyethylene glycol;
the pore-forming agent is Na₂CO₃ or NaHCO₃;
the mass ratio of the chitosan, the superabsorbent polymer monomer, the cross-linking agent, the ammonium persulfate and the pore-forming agent is 1:(100∼300):(0.5∼5):(0.5∼5): (10∼20), and
wherein the chitosan is one or more of a methacrylic anhydride-terminated chitosan which is modified with methacrylic anhydride, an acrylic anhydride-terminated chitosan which is modified with acrylic anhydride, a maleic anhydride-terminated chitosan which is modified with maleic anhydride and an itaconic anhydride-terminated chitosan which is modified with itaconic anhydride.

8. The preparation method according to claim 7, wherein the polyethylene glycol is selected from PEG-400, PEG-600, PEG-1500, PEG-4000, PEG-6000 and PEG-20000.

9. The preparation method according to either of claims 7 or 8, wherein it comprises the following steps: adding the cross-linking agent and the chitosan solution with a concentration of 1%-30% (g/ml) into the superabsorbent polymer monomer aqueous solution with a concentration of (70-99) v% at room temperature, stirring, and then adding the ammonium persulfate, after stirring, adding the pore-forming agent with a concentration of 1%-20% (g/ml), stirring and reacting at 50~100°C for 30-60 minutes to obtain porous sponge.

10. A superabsorbent polymer xerogel sponge, wherein the sponge is prepared by using any of the methods of claims 7-9.

11. Superabsorbent polymer xerogel sponge according to any of claims 1-6 or 10 for use as a hemostatic sponge.

12. Superabsorbent polymer xerogel sponge for the use of claim 11, wherein the hemostatic sponge is used in emergency hemostasis, packing hemostasis, and wound plugging.

## Patentansprüche

1. Superabsorbierender Polymer-Xerogel-Schwamm, der einen porösen Schwamm mit einem dreidimensionalen Netz mit Chitosan als sein Gerüst, superabsorbierendes Polymer als seine verzweigte Kette und Makromolekül oder Polymer mit flexibler Struktur als sein Vernetzungsmittel aufweist, wobei:
das superabsorbierende Polymer Natriumpolyacrylat, Polyacrylamid, Polymethylmethacrylat und/oder Polyvinylalkohol ist,
das Vernetzungsmittel eines oder mehrere ist von: methacrylanyhdridterminiertem Polyethylenglykol, acrylanhydrid-terminiertem Polyethylenglykol, maleinanhydrid-terminiertem Polyethylenglykol und itaconanhydrid-terminiertem Polyethylenglykol, und
das Chitosan eines oder mehrere von einem methacrylanhydrid-terminierten Chitosan, das mit Methacrylanhydrid modifiziert ist, einem acrylanhydrid-terminierten Chitosan, das mit Acrylanhydrid modifiziert ist, einem maleinanhydrid-terminierten Chitosan, das mit Maleinanhydrid modifiziert ist, und einem itaconanhydrid-terminierten Chitosan ist, das mit Icatonanhydrid modifiziert ist.

2. Superabsorbierender Polymer-Xerogel-Schwamm nach Anspruch 1, wobei das Polyethylenglykol ausgewählt ist aus PEG-400, PEG-600, PEG-1500, PEG-4000, PEG-6000 und PEG-20000.

3. Superabsorbierender Polymer-Xerogel-Schwamm nach Anspruch 1 oder 2, wobei das Chitosan 1 ~ 10 % der Gesamtmasse des Schwammes ausmacht, das superabsorbierende Polymer 85 ~ 98 % der Gesamtmasse des Schwammes ausmacht, das Vernetzungsmittel 0,5 ~ 5 % der Gesamtmasse des Schwammes ausmacht.

4. Superabsorbierender Polymer-Xerogel-Schwamm nach Anspruch 1 oder 2, wobei der Schwamm ferner einen Weichmacher, einen Emulgator und/oder ein Antioxidationsmittel umfasst.

5. Superabsorbierender Polymer-Xerogel-Schwamm nach Anspruch 4, wobei der Weichmacher aus Propylenglykol, Glycerin, Sorbitol, Span, Tween, Polyethylenglykol und/oder Natriumfettsäure ausgewählt ist.

6. Superabsorbierender Polymer-Xerogel-Schwamm nach Anspruch 4, wobei der Emulgator aus Span, Tween oder SDS-Lösung mit einer Konzentration von 1 Gew.-% - - 10 Gew.-% ausgewählt ist.

7. Verfahren zur Herstellung eines superabsorbierenden Polymer-Xerogel-Schwamms, wobei das Verfahren umfasst: Mischen von Chitosan, superabsorbierendem Polymermonomer, Vernetzungsmittel, Ammoniumpersulfat und Porosierungsmittel und dann Erhalten des porösen Schwamms durch freie radikale Polymerisationsreaktion, wobei
das superabsorbierende Polymermonomer Natriumacrylat, Acrylamid, Methylmethacrylat und/oder Vinylalkohol ist,
das Vernetzungsmittel eines oder mehrere ist von: methacrylanyhdridterminiertem Polyethylenglykol, acrylanhydrid-terminiertem Polyethylenglykol, maleinanhydrid-terminiertem Polyethylenglykol und itaconanhydrid-terminiertem Polyethylenglykol,
das Porosierungsmittel Na₂CO₃ oder NaHCO₃ ist,
das Massenverhältnis des Chitosans, des superabsorbierenden Polymermonomers, des Vernetzungsmittels, des Ammoniumpersulfats und des Porosierungsmittels 1 : (100 ∼ 300) : (0,5 ∼ 5) : (0,5 ∼ 5) : (10 ∼ 20) beträgt, und
wobei das Chitosan eines oder mehrere von einem methacrylanhydrid-terminierten Chitosan, das mit Methacrylanhydrid modifiziert ist, einem acrylanhydrid-terminierten Chitosan, das mit Acrylanhydrid modifiziert ist, einem maleinanhydrid-terminierten Chitosan, das mit Maleinanhydrid modifiziert ist, und einem itaconanhydrid-terminierten Chitosan ist, das mit Icatonanhydrid modifiziert ist.

8. Herstellungsverfahren nach Anspruch 7 wobei das Polyethylenglykol ausgewählt ist aus PEG-400, PEG-600, PEG-1500, PEG-4000, PEG-6000 und PEG-20000.

9. Herstellungsverfahren nach Anspruch 7 oder 8, wobei es die folgenden Schritte umfasst: Beimengen des Vernetzungsmittels und der Chitosanlösung mit einer Konzentration von 1 % - 30 % (g/ml) in die superabsorbierende wässrige Polymermonomerlösung mit einer Konzentration von (70 - 99) Vol.-% bei Raumtemperatur, Rühren und dann Beimengen des Ammoniumpersulfats, nach dem Umrühren, Beimengen des Porosierungsmittels mit einer Konzentration von 1 % - 20 % (g/ml), Rühren und Reagieren bei 50 ∼ 100 °C während 30 - 60 Minuten zum Erhalten des porösen Schwamms.

10. Superabsorbierender Polymer-Xerogel-Schwamm, wobei der Schwamm unter Verwendung eines der Verfahren nach Anspruch 7 - 9 hergestellt wird.

11. Superabsorbierender Polymer-Xerogel-Schwamm nach einem der Ansprüche 1 - 6 oder 10 zur Verwendung als ein blutstillender Schwamm.

12. Superabsorbierender Polymer-Xerogel-Schwamm zur Verwendung nach Anspruch 11, wobei der blutstillende Schwamm bei der Notfall-Hämostase, Kompressen-Hämostase und beim Wundverschluss verwendet wird.

## Revendications

1. Eponge de xérogel polymère super-absorbant, ayant une éponge poreuse à réseau tridimensionnel avec du chitosane comme son squelette, un polymère super-absorbant comme sa chaîne ramifiée, et une macromolécule ou un polymère avec une structure flexible comme son agent de réticulation, où :
le polymère super-absorbant est du poly(acrylate de sodium), polyacrylamide, poly(méthacrylate de méthyle) et/ou poly(alcool vinylique) ;
l'agent de réticulation est un ou plusieurs parmi : polyéthylèneglycol terminé par un anhydride méthacrylique, polyéthylèneglycol terminé par un anhydride acrylique, polyéthylèneglycol terminé par un anhydride maléique, et polyéthylèneglycol terminé par un anhydride itaconique ; et
le chitosane est un ou plusieurs d'un chitosane terminé par un anhydride méthacrylique qui est modifié avec un anhydride méthacrylique, un chitosane terminé par un anhydride acrylique qui est modifié avec un anhydride acrylique, un chitosane terminé par un anhydride maléique qui est modifié avec un anhydride maléique et un chitosane terminé par un anhydride itaconique qui est modifié avec un anhydride itaconique.

2. Eponge de xérogel polymère super-absorbant selon la revendication 1, où le polyéthylèneglycol est choisi parmi PEG-400, PEG-600, PEG-1500, PEG-4000, PEG-6000, et PEG-20000.

3. Eponge de xérogel polymère super-absorbant selon la revendication 1 ou 2, où le chitosane compte pour 1-10 % de la masse totale de l'éponge ; le polymère super-absorbant compte pour 85~98 % de la masse totale de l'éponge ; l'agent de réticulation compte pour 0,5~5 % de la masse totale de l'éponge.

4. Eponge de xérogel polymère super-absorbant selon la revendication 1 ou 2, où l'éponge inclut de plus un plastifiant, émulsionnant et/ou antioxydant.

5. Eponge de xérogel polymère super-absorbant selon la revendication 4, où le plastifiant est choisi parmi du propylèneglycol, de la glycérine, du sorbitol, Span, Tween, polyéthylèneglycol et/ou acide gras de sodium.

6. Eponge de xérogel polymère super-absorbant selon la revendication 4, où l'émulsionnant est choisi parmi Span, Tween ou une solution SDS avec une concentration de 1 % en masse - 10 % en masse.

7. Procédé de préparation d'une éponge de xérogel polymère super-absorbant, où le procédé comprend : le mélange de chitosane, monomère de polymère super-absorbant, agent de réticulation, persulfate d'ammonium et agent porogène, et puis l'obtention d'éponge poreuse par une réaction de polymérisation radicalaire libre ; où,
le monomère de polymère super-absorbant est l'acrylate de sodium, acrylamide, méthacrylate de méthyle et/ou alcool vinylique ;
l'agent de réticulation est un ou plusieurs parmi : polyéthylèneglycol terminé par un anhydride méthacrylique, polyéthylèneglycol terminé par un anhydride acrylique, polyéthylèneglycol terminé par un anhydride maléique, polyéthylèneglycol terminé par un anhydride itaconique ;
l'agent porogène est Na₂CO₃ ou NaHCO₃ ;
le rapport en masse du chitosane, du monomère de polymère super-absorbant, de l'agent de réticulation, du persulfate d'ammonium et de l'agent porogène est de 1:(100∼300):(0,5∼5):(0,5∼5):(10∼20), et
où le chitosane est un ou plusieurs d'un chitosane terminé par un anhydride méthacrylique qui est modifié avec un anhydride méthacrylique, un chitosane terminé par un anhydride acrylique qui est modifié avec un anhydride acrylique, un chitosane terminé par un anhydride maléique qui est modifié avec un anhydride maléique et un chitosane terminé par un anhydride itaconique qui est modifié avec un anydride itaconique.

8. Procédé de préparation selon la revendication 7, où le polyéthylèneglycol est choisi parmi PEG-400, PEG-600, PEG-1500, PEG-4000, PEG-6000, et PEG-20000.

9. Procédé de préparation selon l'une des revendications 7 et 8, où il comprend les étapes suivantes : addition de l'agent de réticulation et de la solution de chitosane avec une concentration de 1-30% (g/ml) dans la solution aqueuse de monomère de polymère super-absorbant avec une concentration de (70-99)% en volume à température ambiante, agitation, et puis addition du persulfate d'ammonium, après agitation, addition de l'agent porogène avec une concentration de 1%-20% (g/ml), agitation et réaction à 50~100°C pendant 30-60 minutes pour obtenir une éponge poreuse.

10. Eponge de xérogel polymère super-absorbant, où l'éponge est préparée en utilisant l'un quelconque des procédés des revendications 7-9.

11. Eponge de xérogel polymère super-absorbant selon l'une quelconque des revendications 1-6 ou 10 pour une utilisation comme une éponge hémostatique.

12. Eponge de xérogel polymère super-absorbant pour l'utilisation de la revendication 11, où l'éponge hémostatique est utilisée dans une hémostase d'urgence, une hémostase de tamponnement, et un comblement de plaie.
